Europäisches Patentamt

European Patent Office

Office européen des brevets

(19)

(11) Publication number: 0 337 707
A2

# EUROPEAN PATENT APPLICATION

(12)

(21) Application number: 89303525.3

(22) Date of filing: 11.04.89

(51) Int. Cl.4: C12P 13/14

(30) Priority: 12.04.88 JP 90040/88

(43) Date of publication of application:
18.10.89 Bulletin 89/42

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Yamada, Kazuo
1192-227, Shinzaike Hiraoka-cho
Kakogawa Hyogo 675-01(JP)
Inventor: Kagita, Susumu
539-2, Iho 4-chome
Takasago Hyogo 676(JP)
Inventor: Onishi, Masayuki
1023, Saijo Kanno-cho
Kakogawa Hyogo 675(JP)

(74) Representative: Lewin, John Harvey et al
ELKINGTON AND FIFE Beacon House 113
Kingsway
London WC2B 6PP(GB)

(54) Method for purification of polyglutamate.

(57) An improved method for the purification of a PGA salt which comprises adding a hydrophilic organic solvent and a water-soluble salt-to an aqueous solution of a PGA salt containing microbial cells in an amount effective to lower the viscosity of the solution without precipitation of the PGA salt, and then removing the cells.

EP 0 337 707 A2

# METHOD FOR PURIFICATION OF POLYGLUTAMATE

## FIELD OF THE INVENTION

The present invention relates to a method for the purification of a salt of polyglutamic acid (hereinafter, sometimes, abbreviated as PGA) which is expected to be utilized as a functional material in the fields such as food, textile, cosmetics, medicines and the like.

## BACKGROUND OF THE INVENTION

It has been known that PGA or its salt can be produced by a fermentation method utilizing microorganisms (Japanese Patent Publication No 24472/1968). Upon the purification and recovering of PGA or its salt produced, in order to avoid contamination of the product with microbial cells, it is necessary to carry out an operation for removing the cells. However, since a PGA fermented liquid or PGA containing solution has very high viscosity, it is very difficult to remove microbial cells industrially. As a method for lowering the viscosity of an aqueous PGA containing solution, there have been hitherto known (1) a method wherein a fermented liquid or the like having high viscosity is subjected to a heat treatment and then the pH thereof is acidified (Japanese Patent Publication No. 24590/1977), (2) a method wherein the pH of an aqueous PGA solution is adjusted to not higher than 3.5 and then an inorganic salt is added thereto (Japanese Patent Laid Open Publication No. 85687/1973) and the like.

However, in practice, there are problems in the above method. For example, in the case of the above method (1), the characteristic viscosity of the resulting purified and recovered PGA is lowered by acidifying a PGA fermented liquid and, in the case of the above method (2), when the viscosity of a solution to be treated is extremely high, lowering of the viscosity is insufficient and, therefore,it is difficult to efficiently remove microbial cells from a fermented liquid.

## OBJECTS OF THE INVENTION

Under these circumstances, the present inventors have studied a method for efficiently removing microbial cells from an aqueous PGA salt containing solution which contains the cells such as a fermented liquid or the like by lowering the viscosity of the solution with preventing lowering of the inherent viscosity of the product which is characteristic properties of PGA or its salt. As the results, it has been found that addition of a hydrophilic organic solvent and a water-soluble salt to a PGA salt containing solution is effective. Thus, the present invention has been attained.

That is, the main object of the present invention is to provide an improved method for the purification of a PGA salt.

This object as well as other objects and advantages of the present invention will become apparent to those skilled in the art from the following description.

## SUMMARY OF THE INVENTION

According to the present invention, there is provided a method for the purification of a PGA salt which comprises adding a hydrophilic organic solvent and a water-soluble salt to an aqueous solution of a PGA salt containing microbial cells in an amount effective to lower the viscosity of the solution without precipitation of the PGA salt, and then removing the cells.

## DETAILED DESCRIPTION OF THE INVENTION

2

The PGA salt to be purified by the method of the present invention is not limited to a specific one and includes ammonium salt, salts with alkali metals such as sodium, potassium, lithium and the like, and alkaline earth metals such as magnesium, calcium, barium and the like. The preferred PGA salts include ammoium salt and salt with sodium or calcium.

As the above aqueous solution of the PGA salt containing the microbial cells, there are, for example, a PGA salt containing fermented liquid obtained by cultivating a microorganism according to a known method (hereinafter, sometimes, simply abbreviated as a fermented liquid) and a solution of an intermediate product wherein a certain treatment has been conducted to recover PGA or its salt from the fermented liquid (hereinafter, sometimes, abbreviated as a treated solution). It is preferred that the pH of the aqueous solution is near neutral (about 6 to 8).

More particularly, as the treated solution, there is, for example, an aqueous solution of PGA or its salt obtained by adding a hydrophilic organic solvent such as methanol, ethanol or the like and/or a salt such as sodium chloride or the like to the fermented liquid to precipitate PGA or its salt and then re-dissolving it. Although the treated solution contains less microbial cells in comparison with the fermented liquid, the treated solution still contains a considerable amount of the microbial cells.

As the above fermented liquid and treated solution, in general, a solution containing a PGA salt in an amount of not more than about 2.5% (w/w), preferably, not more than about 1.5% (w/w) in terms of PGA can be used.

As the hydrophilic organic solvent used in the present invention, there are, for example, methanol, ethanol, acetone, isopropanol and the like and, particularly, methanol or ethanol is preferable. As the water-soluble salt, there are, for example, sodium chloride, potassium chloride, ammonium acetate, monosodium glutamate, ammonium sulfate and the like and, particularly, sodium chloride is preferred.

Next, the amount of the hydrophilic organic solvent and water-soluble salt to be added to the aqueous solution of a PGA salt is explained.

The amount varies depending upon conditions such as the PGA concentration in-the aqueous solution of a PGA salt, to which solution they being added, i.e., the fermented liquid or the treated solution, kinds of the hydrophilic organic solvent and water-soluble salt to be added and the like. However, for example, the amount can be selected from the range of about 30 to 75% (w/w), preferably about 45 to 60% (w/w), when using methanol as the hydrophilic organic solvent, or about 10 to 50% (w/w), preferably about 20 to 40% (w/w), when using ethanol as the hydrophilic organic solvent, and from the range of about 1 to 5% (w/w), preferably about 1 to 3% (w/w), when using sodium chloride as the water-soluble salt, provided that a PGA salt is not precipitated. It is preferable to add them in such a range that a viscosity of PGA salt solution after addition is generally not more than about 200 cp, particularly, not more than 100 cp.

For example, regarding the aqueous solution containing about 1 to 1.5% of PGA, it is preferable to use sodium chloride in an amount of about 2 to 3% in the case of using 20% ethanol, about 0.5 to 3% in the case of using 30% ethanol, or about 0.5 to 1% in the case of using 40% ethanol. Regarding the aqueous solution containing about 2% of PGA, it is preferable to use sodium chloride in an amount of about 0.5 to 1.5% in the case of using 35% ethanol.

Regarding the aqueous solution containing about 1% of PGA, it is preferable to use ammonium sulfate in an amount of about 0.5 to 3% in the case of using 30% acetone, about 0.1 to 3% in the case of using 35% acetone, or about 0.1 to 1% in the case of using 40% acetone.

Regarding the aqueous solution containing about 1 to 1.5% of PGA, it is preferable to use sodium chloride in an amount of about 1.5 to 3% in the case of using 50% methanol, or about 0.1 to 2.5% in the case of using 60% methanol.

Regarding the aqueous solution containing about 1% of PGA, it is preferable to use calcium chloride in an amount of about 0.01 to 0.1% in the case of using about 50 to 55% methanol, or not more than about 0.06% in the case of using about 60 to 65% methanol.

As the method for addition, it is preferable that, firstly, the water-soluble salt is added to a PGA salt containing solution and dissolved therein and, then, the hydrophilic organic solvent is added thereto. As the operating temperature, generally, it is 0°C to a temperature of not higher than a boiling point of the hydrophilic organic solvent, preferably, about 20 to 50°C.

Thus, the aqueous solution of a PGA salt wherein its viscosity has been reduced is subjected to a separation method which itself has been known such as filtration, centrifugation and the like to remove microbial cells efficiently and, then, the same hydrophilic organic solvent as that described above is added thereto to precipitate the PGA salt. After separating the PGA salt, it is dried to obtain PGA salt powder.

As described hereinabove, according to the method of the purification of the present invention, in comparison with addition of the hydrophilic organic solvent cr the water-soluble salt alone, it is possible to efficiently lower the viscosity of an aqueous solution containing a PGA salt and to efficiently remove microbial cells from an aqueous PGA salt solution containing the cells with less steps. Further, since no acid treatment of a PGA salt is included, it is possible to recover of a PGA salt with preventing lowering of its characteristic viscosity.

The following Examples and Reference Example further illustrate the present invention in detail but are not construed to be limit the scope thereof. In the Examples, all the "% 's" are by weight unless otherwise stated.

In the Examples, the viscosity was measured at room temperature and 60 r.p.m. by using Brookfield rotational viscometer (manufactured by Tokyo Keiki Co., Ltd.).

## Example 1

Sodium chloride was dissolved in a fermented solution (containing 1.3% of PGA, pH 6.5) obtained according to the same manner as that described in Reference Example hereinafter at room temperature so that the concentration as shown in Table 1 was obtained. Then, the ethanol was added and the viscosity of the resulting mixture was measured. The unit of the number in Table 1 is centipoise (cp).

Table 1

| Ethanol | Sodium chloride | | | |
|---|---|---|---|---|
| | 0% | 1% | 2% | 3% |
| 0% | 1316 | 812 | 600 | 480 |
| 10% | 1040 | 590 | 328 | 280 |
| 20% | 760 | 333 | 195 | 163 |
| 30% | 570 | 175 | 61 | 41 |
| 40% | 283 | 37 | * | * |

*: PGA salt was precipitated.

As is clear from the above table, it is possible to efficiently reduce the viscosity of an aqueous solution containing the PGA salt by addition of ethanol in combination with sodium chloride.

## Example 2

Sodium chloride-was dissolved in a fermented solution (containing 1% of PGA, pH 6.5) obtained according to the same manner as that described in Reference Example hereinafter at room temperature so that the concentration as shown in Table 2 was obtained. Then, ethanol was added and the viscosity and the rate of filtration of the resulting mixture were measured, respectively.

In the measurement of the rate of filtration, the filtration was carried out at 25°C under a pressure of 1.8 kg cm² for 60 minutes by using a pressure filter (manufactured by Toyo Seisakusho Co., Ltd.) precoated with 3% of a filter aid Radiolite #200 (manufactured by Showa Chemical Industries Co., Ltd.) per the test solution and the amount of the filtrate was measured as the rate of filtration.

The results are shown in Table 2.

Table 2

| Ethanol (%) | Sodium chloride (%) | Viscosity (cp) | Rate of filtration ($l/m^2hr$) |
|---|---|---|---|
| 0 | 0 | 1060 | could not be measured |
| 0 | 3 | 318 | 8 |
| 10 | 3 | 209 | 48 |
| 20 | 3 | 134 | 102 |
| 30 | 3 | 49 | 154 |

As is clear from the above table, it is possible to efficiently remove microbial cells because the viscosity of the aqueous solution containing the PGA salt is reduced and the rate of filtration is also increased by addition of ethanol in combination with sodium chloride.

In each case, the rate of removal of microbial cells of the filtrate obtained by filtration of the aqueous solution containing the PGA salt was about 98.5 %.

Example 3

Ammonium sulfate was dissolved in a fermented solution (containing 1% of PGA, pH 6.5) obtained according to the same manner as that described in Reference Example hereinafter so that the concentration as shown in Table 3 was obtained. Then, acetone was added and the viscosity of the resulting mixture was measured. The results are shown in the following table.

Table 3

| Acetone | Ammonium sulfate | | | |
|---|---|---|---|---|
| | 0% | 1% | 2% | 3% |
| 0% | 1230 | 816 | 630 | 490 |
| 10% | 936 | 616 | 437 | 363 |
| 20% | 582 | 353 | 278 | 229 |
| 30% | 270 | 167 | 125 | 94 |
| 35% | 156 | 108 | 70 | 47 |
| 40% | 59 | 38 | * | * |

*: PGA was precipitated.

Example 4

To a fermented solution (2 liters, containing 1.0% of PGA, pH 6.5) obtained according to the same manner as that described in Reference Example hereinafter were added sodium chloride (30 g) and ethanol (1.4 liters). After removal of a precipitated viscous mass, water was added to prepare an aqueous solution containing a PGA salt (containing 2% of PGA, pH 6.5). Sodium chloride was dissolved in the resulting aqueous PGA salt containing solution so that the concentration as shown in Table 4 was obtained. Then, ethanol was added and the viscosity (cp) of the resulting mixture was measured.

The results are shown in Table 4.

Table 4

| Ethanol | Sodium chloride | | | |
|---|---|---|---|---|
| | 0% | 1% | 2% | 3% |
| 0% | 5200 | 3900 | 3150 | 3000 |
| 10% | not measured | 2700 | 2100 | 2550 |
| 20% | not measured | 1464 | 1134 | 830 |
| 30% | not measured | 534 | 265 | * |
| 35% | not measured | 155 | * | * |

*: PGA was precipitated.

Example 5

Sodium chloride (60 g) was dissolved in a fermented solution (2 liters, containing 1% of PGA, pH 6.5) at a room temperature and ethanol (1 liter) was added. Then, the mixture was subjected to centrifugation (8000 r.p.m,, 10000 G) to remove microbial cells. Then, ethanol (1 liter) was added to the resulting supernatant (2.5 liters) to precipitate sodium salt of PGA and it was separated with Nutsche covered with Saran woven cloth. After washing it with 80% aqueous ethanol solution, it was dried at 50°C under the reduced pressure to obtain sodium salt of PGA (18 g).

Reference Example

To a commercially available fermented soybeans (natto) was added 5-fold amount of sterilized water and thoroughly mixed and then the solid material was filtered off with a gauze. The filtrate obtained was diluted with sterilized water in turn and 0.1 ml thereof was smeared on a plate culture medium (pH 6.4) containing sucrose (50 g), L-glutamic acid (15 g), $KH_2PO_4$ (2.7 g), $Na_2HPO_4 \cdot 12H_2O$ (4.2 g), $MgSO_4 \cdot 7H_2O$ (0.5 g), NaCl (0.5 g), $MnSO_4 \cdot 4\text{-}6 H_2O$ (2 mg), biotin (100 μg) and agar (15 g) per 1 liter and then incubated at 37°C for 3 days. From the colonies developed, one strain having high viscosity was chosen and, by using the above medium, this was again subjected to single colony separation. The strain capable of producing a viscous material thus obtained was smeared on a slant of L-medium (composed of Bacto-tripton (1%), yeast extract (0.5%), NaCl (1%) and agar (1.5%)) and incubated at 37°C overnight. One loopful of the resulting culture was inoculated in to 200 ml Erlenmeyer flasks into which a medium (pH 6.4) (20 ml) containing glucose (50 g), L-glutamic acid (15 g), $(NH_4)_2SO_4$ (7.0 g), $KH_2PO_4$ (2.7 g), $Na_2HPO_4 \cdot 12H_2O$ (4.2 g). $MgSO_4 \cdot 7H_2O$ (0.5 g), NaCl (0.5 g), $MnSO_4 \cdot 4\text{-}6H_2O$ (2 mg) and biotin (100 μg ) per 1 liter had been distributed and incubated with shaking at 37°C for 2 days to obtain a fermented liquid (pH 6.5).

Example 6

Sodium chloride was dissolved in a fermented solution (containing 1.2% of PGA, pH 6.5) obtained according to the same manner as that described in Reference Example so that the concentration thereof as shown in Table 5 was obtained and, then, methanol was added thereto. The viscosity (cp) of the resulting mixture was measured.

The results are shown in Table 5.

Table 5

| Methanol | Sodium chloride | | | |
|---|---|---|---|---|
| | 0% | 1% | 2% | 3% |
| 0% | 2800 | 2490 | 2120 | 1900 |
| 30% | 1224 | 984 | 764 | 636 |
| 40% | 800 | 580 | 420 | 330 |
| 50% | 385 | 233 | 141 | 95 |
| 60% | 220 | 106 | 46 | * |
| 70% | 72 | * | * | * |

*: PGA was precipitated.

## Example 7

Sodium chloride (60 g) was dissolved in a fermented solution (2 liters, containing 1.2% of PGA, pH 6.4) obtained according to the same manner as that described in Reference Example and methanol (2.6 liters) was added thereto. The mixture was subjected to centrifugation (8000 r.p.m, 10000 G) to remove the microbial cells. Methanol (1.7 liters) was added to the resulting supernatant (4 liters) to precipitate sodium salt of PGA and the salt was separated by Nutsche covered with Saran woven cloth. After washing with 90% and 99% aqueous methanol, the salt was dried at 40° C under reduced pressure to obtain sodium salt of PGA (18 g).

## Example 8

Sodium chloride (60 g) was dissolved in a fermented solution (2 liters, containing 1.3% of PGA, pH 6.5) obtained according to the same manner as that described in Reference Example and, then, methanol (4.6 liters) was added thereto. After separation of the precipitated viscous mass, water was added to prepare an aqueous solution containing the PGA salt (containing 0.8% of PGA, pH 6.4). Calcium chloride was dissolved in the resulting aqueous solution containing the PGA salt so that the concentration as shown in Table 6 was obtained. The viscosity (cp) of the resulting mixture was measured.

The results are shown in Table 6.

Table 6

| Methanol | Calcium chloride | | | |
|---|---|---|---|---|
| | 0% | 0.03% | 0.06% | 0.1% |
| 0% | 1220 | 980 | 800 | 566 |
| 30% | 688 | 496 | 450 | 520 |
| 40% | 360 | 310 | 308 | 310 |
| 45% | 335 | 250 | 240 | 223 |
| 50% | 253 | 185 | 180 | 153 |
| 55% | 215 | 155 | 150 | 75 |
| 60% | 168 | 115 | 100 | * |
| 65% | 98 | 77 | 75 | * |

*: PGA was precipitated.

## Claims

1. A method for the purification of a PGA salt which comprises adding a hydrophilic organic solvent and a water-soluble salt to an aqueous solution of a PGA salt containing microbial cells in an amount effective to lower the viscosity of the solution without precipitation of the PGA salt, and then removing the cells.

2. A method according to claim 1, wherein the hydrophilic organic solvent is selected from the group consisting of methanol, ethanol, acetone and isopropanol.

3. A method according to claim 1, wherein the water-soluble salt is selected from the group consisting of sodium chloride, potassium chloride, ammonium acetate, monosodium glutamate and ammonium sulfate.

4. A method according to claim 1, wherein the organic solvent is methanol and it is added in an amount of about 30 to 75% (w/w).

5. A method according to claim 1, wherein the organic solvent is ethanol and it is added in an amount of about 10 to 50% (w/w).

6. A method according to claim 1, wherein the water-soluble salt is sodium chloride and it is added in an amount of about 1 to 5% (w/w).

7. A method according to claim 1, wherein the organic solvent and the water-soluble salt is added in such an amount that a viscosity of PGA salt solution after, addition becomes not more than about 200 cp.

8. A method according to claim 1, wherein the purification is carried out at a temperature of 0°C to a temperature of not higher than a boiling point of the hydrophilic organic solvent.

9. A method according to claim 1, wherein the PGA salt is ammonium salt, a salt with alkali metal or a salt with alkaline earth metal.

10. A method according to claim 1, wherein the PGA salt is ammonium salt, sodium salt or calcium salt.